(19) Europäisches Patentamt
European Patent Office
Office européen des brevets

(11) **EP 3 049 530 B1**

(12) **EUROPÄISCHE PATENTSCHRIFT**

(45) Veröffentlichungstag und Bekanntmachung des Hinweises auf die Patenterteilung:
**22.06.2022 Patentblatt 2022/25**

(21) Anmeldenummer: **14777515.9**

(22) Anmeldetag: **17.09.2014**

(51) Internationale Patentklassifikation (IPC):
**C12P 21/02** (2006.01)   **C12M 1/00** (2006.01)
**C07K 5/083** (2006.01)

(52) Gemeinsame Patentklassifikation (CPC):
**C07K 5/0808; C07K 5/1016; C07K 5/1021; C12M 23/34; C12M 29/04; C12P 21/02**

(86) Internationale Anmeldenummer:
**PCT/EP2014/002520**

(87) Internationale Veröffentlichungsnummer:
**WO 2015/043729 (02.04.2015 Gazette 2015/13)**

(54) **VERFAHREN ZUR ZELLFREIEN PROTEINSYNTHESE IN GEGENWART EINES CASPASE-INHIBITORS**

METHOD FOR CELL-FREE PROTEIN SYNTHESIS IN THE PRESENCE OF A CASPASE INHIBITOR

PROCÉDÉ DE SYNTHÈSE DE PROTÉINES ACELLULAIRE EN PRÉSENCE D'UN INHIBITEUR DE CASPASES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.09.2013 DE 102013015977**
**11.12.2013 DE 102013020900**

(43) Veröffentlichungstag der Anmeldung:
**03.08.2016 Patentblatt 2016/31**

(73) Patentinhaber: **Fraunhofer-Gesellschaft zur Förderung**
**der angewandten Forschung e.V.**
**80686 München (DE)**

(72) Erfinder:
• **KUBICK, Stefan**
**14109 Berlin (DE)**
• **STECH, Marlitt**
**14476 Potsdam (DE)**
• **WÜSTENHAGEN, Doreen**
**14478 Potsdam (DE)**
• **QUAST, Robert**
**12163 Berlin (DE)**

(74) Vertreter: **v. Bezold & Partner Patentanwälte - PartG mbB**
**Ridlerstraße 57**
**80339 München (DE)**

(56) Entgegenhaltungen:
**WO-A2-99/35277    US-A1- 2006 024 781**

• **MADIN KAIRAT ET AL: "A highly efficient and robust cell-free protein synthesis system prepared from wheat embryos: Plants apparently contain a suicide system directed at ribosomes", PROCEEDINGS OF THE NATIONAL ACADEMY OF SCIENCES, NATIONAL ACADEMY OF SCIENCES, US, Bd. 97, Nr. 2, 18. Januar 2000 (2000-01-18), Seiten 559-564, XP002286586, ISSN: 0027-8424, DOI: 10.1073/PNAS.97.2.559 in der Anmeldung erwähnt**
• **SPIRIN A S ET AL: "A CONTINUOUS CELL-FREE TRANSLATION SYSTEM CAPABLE OF PRODUCING POLYPEPTIDES IN HIGH YIELD", SCIENCE, AMERICAN ASSOCIATION FOR THE ADVANCEMENT OF SCIENCE, US, Bd. 242, 25. November 1988 (1988-11-25), Seiten 1162-1164, XP002044225, ISSN: 0036-8075, DOI: 10.1126/SCIENCE.3055301 in der Anmeldung erwähnt**

• HELMUT MERK: "Cell-free synthesis of functional and endotoxin-free antibody Fab fragments by translocation into microsomes", BIOTECHNIQUES, Bd. 53, 1. September 2012 (2012-09-01), Seiten 153-160, XP055043178, DOI: 10.2144/0000113904

**Beschreibung**

Die Erfindung betrifft ein verbessertes Verfahren zur zellfreien Proteinsynthese unter Verwendung eines eukaryotischen Zelllysats in Gegenwart eines Caspase-Inhibitors, und die Verwendung eines Caspase-Inhibitors zur Steigerung der Proteinausbeute in einem solchen zellfreien Verfahren zur Proteinsynthese unter Verwendung eines eukaryotischen Zelllysats.

Hintergrund der Erfindung

In der jüngeren Vergangenheit hat sich die zellfreie Proteinsynthese als effiziente Alternative zur Expression von Proteinen *in vivo* etabliert (Carlson, E.D., et al., Biotechnology Advances, 2012, 30(5): S. 1185-1194). Hierbei werden die Inhaltsstoffe der Zelle genutzt, um ein bestimmtes Zielprotein auf schnelle, zuverlässige und kostengünstige Weise herzustellen. Die gewonnenen Zellextrakte, die auch als Zelllysate bezeichnet werden, enthalten die essentiellen Komponenten, die für die zellfreie Synthese von Proteinen benötigt werden: Ribosomen, Translationsfaktoren und Enzyme. Inzwischen können ausgewählte rekombinante Proteine in prokaryotischen sowie eukaryotischen Zelllysaten in funktionell aktiver Form hergestellt werden. Folgende Translationssysteme auf Basis von eukaryotischen Zelllysaten werden zurzeit verstärkt angewendet: Weizenkeimlysate, Retikulozytenlysat, Insektenzelllysate sowie Zellextrakte aus HeLa- und -Hybridom-Zellen.

Im Vergleich zu prokaryotischen *in vitro* Translationssystemen basierend auf *Escherichia coli* sind die erzielten Proteinausbeuten unter Anwendung der meisten eukaryotischen Translationssysteme verhältnismäßig gering (Carlson et al., oben). Eine Ausnahme bildet hier das äußerst leistungsfähige Weizenkeimlysat-Expressionssystem. Dieses erzielt je nach Protein und Reaktionsformat mehrere hundert Mikrogramm Protein je Milliliter Reaktionsvolumen (Madin, K., et al., Proc. Natl. Acad. Sci. USA, 2000. 97(2): S. 559-64). Allerdings ist dieser Zellextrakt nicht geeignet, um Proteine mit posttranslationalen Modifizierungen wie zum Beispiel Glykosylierungen zu synthetisieren.

Eukaryotische Translationssysteme basierend auf Insektenzell- und Retikulozytenlysaten erlauben die Synthese von komplex aufgebauten, eukaryotischen Proteinen mit posttranslationalen Modifizierungen, die in *Escherichia coli* nicht synthetisiert werden können. Retikulozytenlysate müssen jedoch zu diesem Zweck mit mikrosomalen Membranen einer anderen Spezies angereichert werden (heterogenes Translationssystem). Im Gegensatz dazu können Zelllysate aus *Spodoptera frugiperda* als homogenes Translationssystem verwendet werden, da das Zelllysat und die enthaltenen Membranvesikel aus derselben Zelllinie gewonnen werden. Durch geeignete Zellaufschlussmethoden können eukaryotische Zelllysate gewonnen werden, die Bestandteile eines wichtigen zellulären Kompartiments, des sogenannten endoplasmatischen Retikulums, enthalten. Alle Proteine, die aus der Zelle ausgeschleust oder in die Zellmembran eingebaut werden, Zuckerreste tragen oder Disulfidbrücken zur Stabilisierung ihrer Molekülstruktur besitzen, wandern durch dieses zelluläre Kompartiment. Zelllysate, die Strukturen des ER besitzen, sogenannte Mikrosomen oder Membranvesikel, können nun genutzt werden, um eben solche Proteinkandidaten in funktionell aktiver Form herzustellen. Auf diese Weise werden Unverträglichkeiten zwischen den Vesikeln und den zytosolischen Proteinen des Lysates vermieden, was relativ hohe Proteinausbeuten (bis 20 $\mu$g/ml in Insektenzelllysaten im Batch-Modus) und einen effizienten Transport der Zielproteine in die Mikrosomen des Lysates zur Folge hat.

Das Dokument US 2006/024781 A1 beschreibt ein Verfahren zur Detektion und massenspektrometrischen Analyse von neu gebildeten Proteinen, insbesondere verkürzten Proteinen, welche bevorzugt in einem Verfahren zur zellfreien Proteinsynthese hergestellt werden. Eine Ausführungsform des Verfahrens beinhaltet die Zugabe von Proteaseinhibitoren zur Verhinderung des proteolytischen Abbaus der gebildeten Proteine.

Zellfreie Proteinsynthese-Reaktionen können auf verschiedene Weise experimentell umgesetzt werden. Die einfachste Reaktionsführung ist die Synthese eines Zielproteins in einer "Eintopfreaktion" (Batch-Reaktion). Batchbasierte Systeme eignen sich daher zur unkomplizierten und schnellen Synthese eines Zielproteins. Auf der anderen Seite sind sie jedoch durch kurze Laufzeiten und relativ geringe Proteinausbeuten gekennzeichnet.

In der Regel erreicht eine Batch-basierte zellfreie Translationsreaktion nach 1-1,5 h das Maximum an synthetisiertem Zielprotein. Darüber hinausgehende Inkubationszeiten führen nicht zu gesteigerten Proteinausbeuten, sondern resultieren mit großer Wahrscheinlichkeit in einer Abnahme der Konzentration des Zielproteins, möglicherweise bedingt durch den proteolytischen Abbau der Zielproteine, zum Beispiel durch im Zellextrakt vorhandene endogene Proteasen. Es wäre jedoch von großem Vorteil, in bestimmten Fällen die synthetisierten Zielproteine auch über längere Inkubationszeiten (> 2 h) hinweg in intakter Form im Translationsansatz zu erhalten.

Eine Möglichkeit, die Laufzeit einer zellfreien Proteinsynthese-Reaktion zu verlängern, um somit höhere Proteinausbeuten zu erzielen, ist die Verwendung von Dialysesystemen *(continuous exchange cell-free systems,* CECF; Spirin, A., et al., Science, 1988, 242 (4882) : S. 1162-4). Hierbei gelangen energiereiche Substanzen wie ATP und GTP durch Diffusion über eine Membran in das Reaktionskompartiment, den Ort der Translation. Gleichzeitig wird die Reaktion von inhibierenden Substanzen wie freien Phosphaten und ADP abgereichert.

Die kontinuierliche Versorgung der Zellfrei-Reaktion im Reaktionskompartiment verlängert die Laufzeit der Synthese und führt zu deutlich gesteigerten Proteinausbeuten im Vergleich zum Batch-System. Solche Dialyse-Systeme sind bereits im Handel erhältlich, allerdings ausschließlich in Kombination mit prokaryotischen Zelllysaten aus *Escherichia coli* oder mit Weizenkeimlysaten.

Trotz der Ausbeutesteigerung gegenüber diskontinuierlichen Batch-Systemen ist die Proteinausbeute bei entsprechenden eukaryotischen Dialyse-Systemen im Vergleich zu einem *Escherichia coli* basierten System immer noch relativ gering. Vor diesem Hintergrund besteht die Aufgabe der Erfindung in der Bereitstellung von Mitteln, um die Synthese von komplexen eukaryotischen und prokaryotischen Proteinen in stabiler und funktionell aktiver Form, insbesondere auch Membranproteine bzw. Proteine mit posttranslationalen Modifizierungen, in großer Menge in einem zellfreien Translationssystem zu ermöglichen.

Diese Aufgabe wird erfindungsgemäß gelöst durch das Verfahren zur zellfreien Proteinsynthese nach Anspruch 1, bei dem ein eukaryotisches Zelllysat verwendet und die Translationsreaktion in Gegenwart eines Caspase-Inhibitors durchgeführt wird, und die Verwendung eines Caspase-Inhibitors nach Anspruch 7 Speziellere oder bevorzugte Ausführungsformen und Aspekte der vorliegenden Erfindung sind Gegenstand der abhängigen Ansprüche.

Beschreibung der Erfindung

Ein Hauptaspekt der vorliegenden Erfindung betrifft ein Verfahren zur zellfreien Proteinsynthese, welches eine *in vitro*-Translationsreaktion unter Verwendung einer Nukleinsäure-Matrize und eines eukaryotischen Zelllysats umfasst, dadurch gekennzeichnet, dass die Translationsreaktion in Gegenwart eines Caspase-Inhibitors durchgeführt wird und das Verfahren in einer Vorrichtung durchgeführt wird, die mindestens zwei durch eine Dialysemembran getrennte Kompartimente umfasst, wobei die Translationsreaktion in mindestens einem ersten Kompartiment, dem Reaktionskompartiment, stattfindet und während der Translationsreaktion durch die Dialysemembran i) Reaktanden aus mindestens einem weiterem Kompartiment, dem Ver- und Entsorgungskompartiment, in das Reaktionskompartiment diffundieren und ii) Reaktions-nebenprodukte aus dem Reaktionskompartiment in das Ver- und Entsorgungskompartiment diffundieren.

Das verwendete eukaryotische Zelllysat ist nicht besonders beschränkt und umfasst grundsätzlich jedes Zelllysat, welches alle zur in *vitro* Translation der verwendeten Nukleinsäure-Matrize erforderlichen Komponenten enthält. Besonders bevorzugt ist ein Zelllysat, welches auch die Synthese von komplexen Proteinen mit posttranslationalen Modifikationen erlaubt. In einer spezielleren Ausführungsform ist das verwendete eukaryotische Zelllysat aus der Gruppe, die Weizenkeimlysate, Insektenzelllysate, insbesondere Sf21-Zelllysate, Retikulozytenlysate, Keratinocytenlysate, Zellextrakte aus CHO-Zellen, HeLa-Zellen, Hybridomzellen oder kultivierten Lymphomzellen, umfasst, ausgewählt.

Diese Zelllysate können in ihrer nativen Form verwendet oder durch Hinzufügung oder Entfernung bestimmter Komponenten modifiziert werden.

Falls das primär aus eine Zelllinie erhaltene Zelllysat beispielsweise keine Membranvesikel enthält (wie z.B. bei Retikulozytenlysaten), können diese aus einer anderen Quelle, z.B. dem Lysat einer anderen Zelllinie, hinzugefügt werden, um auch die Synthese von Proteinen mit posttranslationalen Modifikationen zu ermöglichen. Desweiteren ist das Vorhandensein der membranären Vesikel im Lysat die Voraussetzung für die Einbettung von Membranproteinen in eine native Lipid-Protein-Matrix, die ihre korrekte Faltung und Konformation gewährleistet.

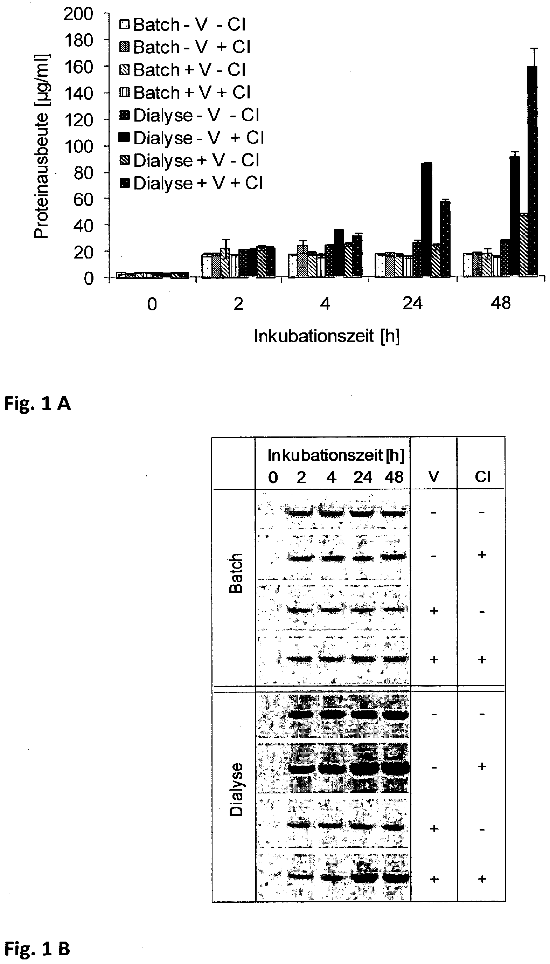

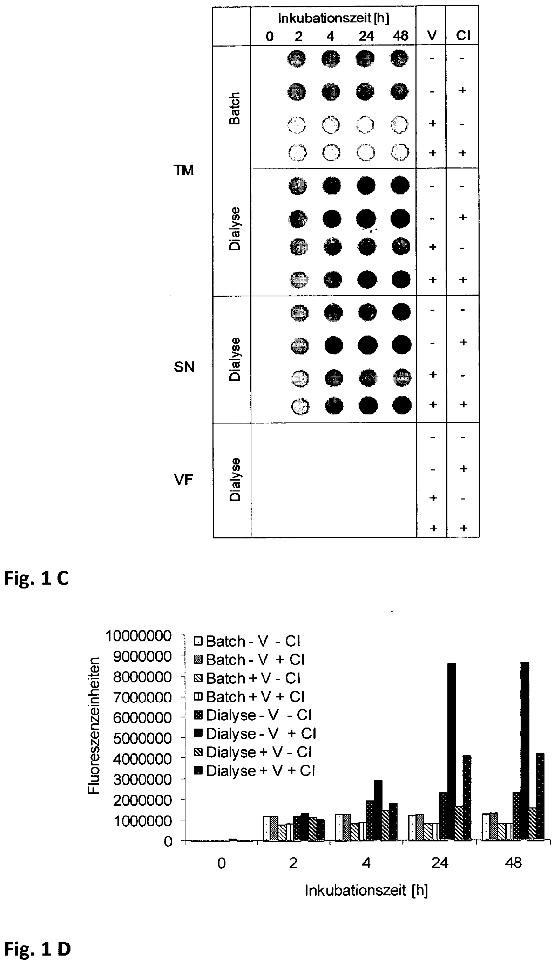

Umgekehrt kann es vorteilhaft sein, zur Synthese bestimmter Proteine ein Zelllysat zu verwenden, das natürlicherweise keine Membranvesikel enthält oder das von Vesikeln befreit wurde, z.B. durch einen Zentrifugationsschritt. Die Verwendung dieses "vesikelabgereicherten" Zelllysates kann für bestimmte Proteine zu einer Ausbeutesteigerung führen (siehe Beispiel 1, Fig. 1).

Das "vesikelabgereicherte" Zelllysat ist ebenso translationsaktiv und kann zur zellfreien Synthese von Proteinen ohne posttranslationale Modifizierungen verwendet werden.

Die Komponenten des verwendeten Zelllysats können somit aus Zellen einer Zelllinie (homogenes Translationssystem) oder von verschiedenen Zelllinien (heterogenes Translationssystem) stammen. Auch die Verwendung artifizieller Zelllysate, bei denen eine oder mehrere Komponenten synthetisch erzeugt wurden, ist grundsätzlich möglich.

Die Reaktionsmischung zur Durchführung des erfindungsgemäßen Verfahrens enthält neben dem eukaryotischen Zelllysat und dem Caspase-Inhibitor noch mindestens eine Nukleinsäure-Matrize, eine Polymerase, Aminosäuren sowie energiereiche Substanzen wie ATP, GTP etc. Prinzipiell können alle für *in vitro*-Translationssysteme bekannten Reaktionsmischungen und Komponenten (nach Zusatz des Caspase-Inhibitors) eingesetzt werden. Die Reaktionsmischung kann gegebenenfalls auch weitere Zusätze, welche die Synthese oder Stabilität bestimmter Zielproteine unterstützen, z.B. DTT oder andere Reduktionsmittel, insbesondere Mischungen aus reduziertem und oxidiertem Glutathion, enthalten.

Wie die im Folgenden beschriebenen Experimente und Versuchsdaten belegen, ermöglicht der Zusatz eines Caspase-Inhibitors zu einem zellfreien eukaryotischen Translationssystem insbesondere unter Anwendung von kontinuierlichen Dialyse-Systemen überraschenderweise eine erhebliche Steigerung der Proteinausbeute, was auch für komplexe Proteine mit posttranslationalen Modifikationen gezeigt wurde.

Der biochemische Mechanismus für diese Wirkung eines Caspase-Inhibitors auf die Syntheseleistung der zellfreien Proteinsynthese-Reaktion ist derzeit noch ungeklärt. Während des Zellaufschlusses werden die Zellen großem Stress ausgesetzt, der möglicherweise apoptotische Prozesse induziert. Die Apoptose wird auch als "programmierter Zelltod" bezeichnet, der maßgeblich von einer Enzymklasse mit proteolytischer Aktivität gesteuert wird, den sogenannten Caspasen. Es wird vermutet, dass die Inhibierung der Caspasen durch den Caspase-Inhibitor die Lebensdauer bestimmter Translationsfaktoren im Lysat verlängert, was sich wiederum positiv auf die Gesamtsyntheseleistung des Zellextraktes auswirken könnte.

Ein weiterer Vorteil der Gegenwart eines Caspase-Inhibitors in einem zellfreien Proteinsynthese-System unter Verwendung eines eukaryotischen Zelllysats besteht in einem positiven Einfluss auf die Stabilität der synthetisierten Proteine, insbesondere unter Bedingungen bei denen diese Proteine länger (z.B. > 1,5 h) im Translationsansatz verbleiben. Dies betrifft zum Beispiel Reaktionen, in denen die zellfreie Synthese eines oder mehrerer Zielproteine in mehreren aufeinanderfolgenden Synthesen in den gleichen Mikrosomensatz erfolgt. Diese Art der Reaktionsführung wird als "Mehrfach- oder Repetitiv-Synthese" bezeichnet und dient der Anreicherung *de novo* synthetisierter Zielproteine im Lumen oder der Membran von mikrosomalen Vesikeln.

Als Caspase-Inhibitor ist grundsätzlich jeder irreversible oder reversible Inhibitor einer Caspase, insbesondere einer der bisher bekannten Caspase-Typen 1-14, geeignet. Caspase-Inhibitoren werden beispielsweise in den folgenden Veröffentlichungen beschrieben: Callus, B.A. and D.L. Vaux, Caspase inhibitors: viral, cellular and chemical. Cell Death Differ, 2006. 14(1): p. 73-78.; Ekert, P.G., Silke, J., Vaux, D.L., Caspase inhibitors. Cell Death Differ, 1999. 6: p. 1081-1086.

Eine spezielle Ausführungsform des erfindungsgemäßen Verfahrens ist dadurch gekennzeichnet, dass der Caspase-Inhibitor ein Aminosäure- oder Peptidderivat ist, umfassend eine Aminosäure oder Peptidsequenz, die als Substrat für eine Caspase, insbesondere eine oder mehrere der Caspase-Typen 1-14, dient, sowie eine funktionelle Gruppe, welche irreversibel oder reversibel an eine Caspase, insbesondere eine oder mehrere der Caspase-Typen 1-14, bindet. Der Caspase-Inhibitor ist hier ein kompetitiver Inhibitor.

Spezieller ist das Verfahren dadurch gekennzeichnet, dass der Caspase-Inhibitor die Aminosäure Aspartat oder eine Peptidsequenz, welche die Aminosäure Aspartat enthält, umfasst. Caspasen spalten Peptidbindungen C-terminal von Aspartat (D). Daher ist die Aminosäure Aspartat in jedem kommerziellen peptidbasierten Caspase-Inhibitor enthalten.

Noch spezieller ist die Aminosäure oder Peptidsequenz aus der Gruppe aus Aspartat, Valin-Alanin-Aspartat (VAD), Aspartat-Glutamat-Valin-Aspartat (DEVD; SEQ-ID-Nr. 1) und Tyrosin-Valin-Alanin-Aspartat (YVAD; SEQ-ID-Nr.2) ausgewählt. Ein Inhibitor mit der Sequenz VAD ist bevorzugt, da es sich dabei um einen generellen Caspase-Inhibitor handelt.

Inhibitoren mit einem anderen Peptid wie zum Beispiel Ac-DEVD-CMK (inhibiert Caspase 3, 6, 7, 8, 10), Z-WEHD-FMK (SEQ-ID-Nr. 3; Caspase 1), Z-AEVD-FMK (SEQ-ID-Nr. 4; Caspase 10), Z-LEED-FMK (SEQ-ID-Nr. 5; Caspase 13), Z-VDVAD-FMK (SEQ-ID-Nr. 6; Caspase 2), Z-DEVD-FMK (Caspase 3), Z-YVAD-FMK (Caspase 4), Z-VEID-FMK (SEQ-ID-Nr. 7; Caspase 6), Z-IETD-FMK (SEQ-ID-Nr. 8; Caspase 8), Z-LEHD-FMK (SEQ-ID-Nr. 9; Caspase 9) sind jedoch ebenfalls einsetzbar.

Die funktionelle Gruppe kann prinzipiell jede Gruppe sein, welche irreversibel oder reversibel an das aktive Zentrum einer Caspase bindet und deren Aktivität blockiert. Verschiedene solcher Gruppen sind bereits für andere Enzyme bekannt und der Fachmann kann geeignete Vertreter dieser Gruppen unschwer durch Routine-Hemmexperimente identifizieren.

Die funktionelle Gruppe des Inhibitors definiert dessen Wirkmechanismus. Peptide, die an die funktionelle Gruppe Methylketon (z.B. Fluormethylketon (FMK), Chlormethylketon (CMK), Acylmethylketon und (Phosphinyloxy)methylketon) gekoppelt sind, wirken als irreversible Inhibitoren (z.B. Z-VAD-FMK, Ac-VAD-CMK, Ac-DEVD-CMK). Peptide, die an Aldehyde (oder Nitrile und Ketone) gekoppelt sind, wirken als reversible Inhibitoren (z.B. Ac-AAVALLPAVLLALLAPDEVD-CHO (SEQ-ID-Nr. 10) oder andere reversible Inhibitoren mit der Aminosäure Aspartat oder der Sequenz DEVD oder VAD).

In einer speziellen Ausführungsform der Erfindung umfasst der Caspase-Inhibitor die funktionelle Gruppe Fluormethylketon (FMK), Chlormethylketon (CMK) oder Difluorphenoxymethylketon, welche irreversibel an alle Caspase-Typen 1-14 bindet.

In einer anderen speziellen Ausführungsform der Erfindung ist der Caspase-Inhibitor ein an mindestens eine Aldehydgruppe gekoppeltes Peptid, das reversibel eine Caspase inhibiert (z.B. Ac-AAVALLPAVLLALLAPDEVD-CHO). Das Peptid hat vorzugsweise eine der oben angegebenen Sequenzen.

Die Konzentration des Caspase-Inhibitors kann je nach Art des verwendeten Zelllysats und der Art und Wirkungsweise des verwendeten Inhibitors stark variieren. Die optimale Konzentration kann jedoch unschwer durch Routineexperimente vom Fachmann ermittelt werden.

Typischerweise liegt der Caspase-Inhibitor in einer Konzentration von 20 $\mu$M bis 100 $\mu$M, vorzugsweise 25 bis 50 $\mu$M, z.B. etwa 30 $\mu$M, in der Reaktionsmischung vor.

Das erfindungsgemäße Verfahren wird kontinuierlich in einem an sich bekannten Dialyse-System durchgeführt, und zwar in einer Vorrichtung , die mindestens zwei durch eine Dialysemembran getrennte Kompartimente umfasst, wobei die Translationsreaktion in mindestens einem ersten Kompartiment, dem Reaktionskompartiment, stattfindet und während der Translationsreaktion durch die Dialysemembran i) Reaktanden aus mindestens einem weiterem Kompartiment, dem Ver- und Entsorgungskompartiment, in das Reaktionskompartiment diffundieren und ii) Reaktionsnebenprodukte aus dem Reaktionskompartiment in das Ver- und Entsorgungskompartiment diffundieren.

Der Caspase-Inhibitor liegt dabei erfindungsgemäß mindestens im Reaktionskompartiment vor, kann jedoch vorzugsweise auch im Ver- und Entsorgungskompartiment vorliegen, um unverbrauchten Inhibitor an das Reaktionskompartiment nachzuliefern.

Die Anwesenheit des Caspase-Inhibitors in der Reaktionsmischung erlaubt wesentlich längere Laufzeiten der Reaktion (z.B. bis zu 48 h oder noch länger) und führt damit auch zu deutlichen Proteinausbeutesteigerungen.

Mit einem solchen kontinuierlichen Verfahren kann bei erfindungsgemäßer Verwendung eines Caspase-Inhibitors auch für komplexe Proteine mit posttranslationalen Modifizierungen eine maximale Proteinausbeute von mindestens 30 µg/ml Reaktionsmedium, vorzugsweise mindestens 100 µg/ml oder 150 µg/ml, erzielt werden.

Ein eng verwandter Aspekt der vorliegenden Erfindung betrifft daher die Verwendung eines Caspase-Inhibitors zu Steigerung der Proteinausbeute in einem zellfreien kontinuierlichen Verfahren zur Proteinsynthese unter Verwendung eines eukaryotischen Zelllysats, wobei das Verfahren in dem oben beschriebenen Dialyse-System durchgeführt wird.

Vorzugsweise ist dieser Caspase-Inhibitor ein Inhibitor wie oben definiert.

Konkreter ist diese Verwendung dadurch gekennzeichnet, dass die maximale Proteinausbeute, gemessen in µg/ml Reaktionsmedium, um einen Faktor von mindestens 2, insbesondere mindestens 5 oder 10, im Vergleich zum analogen System in Abwesenheit des Caspase-Inhibitors gesteigert wird. Dabei werden Proteinausbeuten von mindestens 30 µg/ml Reaktionsmedium, vorzugsweise mindestens 100 µg/ml oder 150 µg/ml, auch bei komplexen Proteinen mit posttranslationalen Modifikationen erzielt.

Ein weiterer verwandter Aspekt, welcher jedoch nicht Gegenstand der beanspruchten Erfindung ist, betrifft die Verwendung eines Caspase-Inhibitors zu Erhöhung der Stabilität der synthetisierten Proteine in einem zellfreien Verfahren zur Proteinsynthese unter Verwendung eines eukaryotischen Zelllysats, insbesondere unter Bedingungen bei denen diese Proteine längere Zeit, z.B. > 1,5 h, im Translationsansatz verbleiben.

Vorzugsweise ist dieser Caspase-Inhibitor ein Inhibitor wie oben definiert, besonders bevorzugt ein irreversibler Inhibitor wie oben definiert.

In einer speziellen Ausführungsform ist diese Verwendung dadurch gekennzeichnet, dass das zellfreie Verfahren zur Proteinsynthese mindestens die folgenden Schritte umfasst:

a) Synthese des Zielproteins mittels einer *in vitro* Translationsreaktion in einem Reaktionsmedium, umfassend eine Nukleinsäure-Matrize, die für das Zielprotein codiert, ein Zelllysat, das Membranvesikel enthält, sowie einen CaspaseInhibitor;

b) Abtrennen von Membranvesikeln, welche das synthetisierte Zielprotein enthalten, von dem Medium;

c) Überführen der abgetrennten Membranvesikel in ein sekundäres Reaktionsmedium, umfassend eine Nukleinsäure-Matrize, die für das Zielprotein codiert, ein Zelllysat, das keine Membranvesikel enthält, sowie einen Caspase-Inhibitor, Durchführen einer *in vitro* Translationsreaktion in Gegenwart des Caspase-Inhibitors in dem sekundären Reaktionsmedium, und Abtrennen von Membranvesikeln, die eine erhöhte Menge des synthetisierten Zielproteins enthalten, von den sekundären Medium, wobei der Schritt c) einmal oder mehrmals wiederholt werden kann.

Weiterhin offenbart, jedoch nicht Gegenstand der beanspruchten Erfindung, ist eine Vorrichtung zur Durchführung des erfindungsgemäßen Verfahrens zur zellfreien Proteinssynthese.

Typischerweise umfasst die Vorrichtung zur Durchführung einer zellfreien Proteinsynthese mindestens zwei verschiedene getrennte Kompartimente:

- mindestens ein Reaktionskompartiment, in dem eine *in vitro*-Translationsreaktion stattfindet und welches die Reaktionsmischung enthält, die mindestes ein eukaryotisches Zelllysat, eine Polymerase, eine Nukleinsäure-Matrize, Aminosäuren, sowie energiereiche Substanzen wie ATP, GTP etc. umfasst;
- mindestens ein von dem Reaktionskompartiment durch eine semipermeable Dialysemembran getrenntes Ver- und Entsorgungskompartiment, welches Aminosäuren, energiereiche Substanzen und Reaktionsnebenprodukte umfasst;

und ist dadurch gekennzeichnet, dass mindestens das Reaktionskompartiment, vorzugsweise auch das Ver- und Entsorgungskompartiment, einen Caspase-Inhibitor wie oben definiert enthält.

Die mit dem erfindungsgemäßen Verfahren synthetisierten Proteine können sowohl prokaryotische als auch eukaryotische Proteine sein. Besonders bevorzugt handelt es sich dabei um Membranproteine bzw. komplexe Proteine mit posttranslationalen Modifizierungen. Solche Modifizierungen können beispielsweise Disulfidbrücken, Glykosylierungen, Lipidmodifizierungen und andere bekannte Modifizierungen sein.

Das hier beschriebene Translationssystem unter Anwendung eines eukaryotischen Zelllysats und Zusatz des Caspase-Inhibitors stellt einen erheblichen Fortschritt auf dem Gebiet der rekombinanten Proteinexpression dar. Sowohl präparative als auch analytische Anwendungen für die hergestellten Proteine werden durch die erzielten Ausbeutesteigerungen bzw. Stabilitätserhöhungen deutlich erleichtert und effizienter. Besonders im Bereich der Expression von Membranproteinen zur Aufklärung von Proteinstrukturen ist dies von großem Interesse.

KURZBESCHREIBUNG DER FIGUREN

**Fig.** 1 Einfluss des Caspase-Inhibitors (CI) Z-VAD-FMK (Benzyloxycarbonyl-Val-Ala-Asp(O-Methyl)-Fluormethylketon) auf die Synthese des zytosolischen Proteins SII-eYFP in einem zellfreien eukaryotischen Translationssystem auf Basis eines Zellextraktes aus *Spodoptera frugiperda (Sf21)* Insektenzellen.

A: Grafische Darstellung der über den Einbau von $^{14}$C-Leucin ermittelten Proteinausbeute von SII-eYFP.

B: Autoradiogramm zur Darstellung von $^{14}$C-Leucin-markiertem SII-eYFP. Das Protein zeigt eine apparente molekulare Masse von ca. 29 kDa.

C: Analyse der Fluoreszenzintensität des zellfrei synthetisierten Proteins SII-eYFP.

D: Grafische Darstellung der unter C erhaltenen Fluoreszenzintensitäten. TM = Gesamttranslationsansatz. SN = Überstand nach Zentrifugation. VF = Vesikuläre Fraktion. V = Vesikel.

**Fig.** 2 Einfluss des Caspase-Inhibitors (CI) Z-VAD-FMK auf die Expression von Membranproteinen in einem zellfreien eukaryotischen Translationssystem auf Basis eines Zellextraktes aus *Spodoptera frugiperda (Sf21)* Insektenzellen.

A: Grafische Darstellung der im Batch- und Dialyse-System erzielten Proteinausbeuten.

B: Autoradiogramm der im Batch (B)- und Dialyse-System (D) synthetisierten Proteine. Kontrolle = Translationsansatz ohne Zugabe einer DNA-Matrize.

**Fig.** 3 Einfluss des reduzierenden Agenz Dithiothreitol (DTT) auf die Expression verschiedener Modellproteine im zellfreien eukaryotischen Translationssystem auf Basis eines Zellextraktes aus *Spodoptera frugiperda (Sf21)* Insektenzellen. Die Abbildung zeigt das Autoradiogramm der im Batch- und Dialyse-System synthetisierten Proteine. Kontrolle = Translationsansatz ohne Zugabe einer DNA-Matrize.

**Fig.** 4 Expression des Typ-I-Transmembranproteins Mel-Hb-EGF-eYFP im zellfreien eukaryotischen Translationssystem auf Basis eines Zellextraktes aus *Spodoptera frugiperda (Sf21)* Insektenzellen.

A: Synthese von Mel-Hb-EGF-eYFP im Batch (links)- und Dialyse-System (rechts) in Abwesenheit (-) und in Gegenwart (+) von DTT unter Zusatz des Caspase-Inhibitors Z-VAD-FMK.

B: Grafische Darstellung der über den Einbau von $^{14}$C-Leucin ermittelten Proteinausbeuten von Mel-Hb-EGF-eYFP im Translationsmix (TM) und der vesikulären Fraktion (VF) über einen Zeitraum von 48 h im Batch- (links) und Dialyse-System (rechts).

C: Autoradiogramm zur Darstellung von $^{14}$C-Leucin markiertem Mel-Hb-EGF-eYFP. Das Protein zeigt eine apparente molekulare Masse von ca. 51 kDa.

```
Kontrolle = Translationsansatz ohne Zugabe einer DNA-Matrize.
```

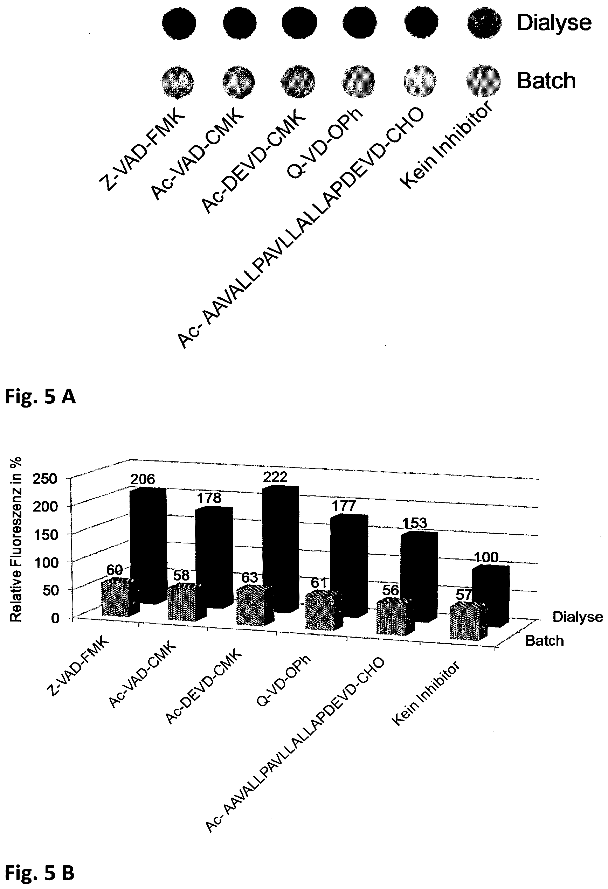

**Fig.** 5 Einfluss irreversibler Caspase-Inhibitoren (Z-VAD-FMK, Ac-VAD-CMK, Ac-DEVD-CMK, Q-VD-OPh) und eines reversiblen Caspase-Inhibitors (Ac-AAVALLPAVLLALLAPDEVD-CHO) auf die Synthese des fluoreszierenden Proteins SII-eYFP in einem eukaryotischen Translationssystem auf Basis eines Zellextraktes aus *Spodoptera frugiperda (Sf21)* Insektenzellen. Ac-VAD-CMK = Acetyl-Val-Ala-Asp-Chlormethylketon. Ac-DEVD-CMK = Acetyl-Asp-Glu-Val-Asp-Chlormethylketon. Q-VD-OPh = N-(2-Quinolyl)-Val-Asp-(2,6-Difluorphenoxy)-Methylketon. Ac-AAVALLPAVLLALLAPDEVD-CHO = Acetyl-Ala-Ala-Val-Ala-Leu-Leu-Pro-Ala-Val-Leu-Leu-Ala-Leu-Leu-Ala-Pro-Asp-Glu-Val-Asp-Aldehyd.

A: Darstellung der relativen Fluoreszenzintensitäten aller Proben im Batch- und Dialyse-Modus.

B: Grafische Auswertung der relativen Fluoreszenzintensität von SII-eYFP im Batch- und Dialyse-Modus. Die Fluoreszenzintensitäten der Proben wurden normiert, wobei die Fluoreszenzintensität des Dialyse-Ansatzes ohne Zusatz eines Caspase-Inhibitors 100% entspricht.

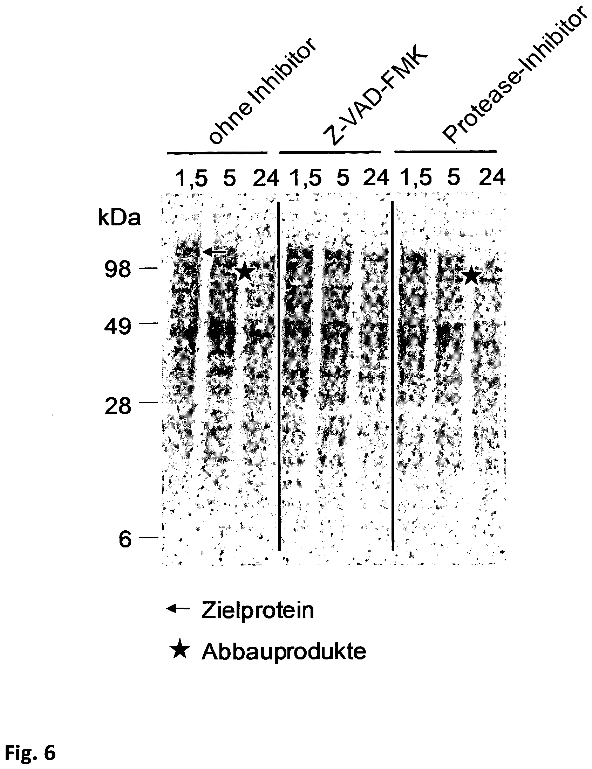

**Fig.** 6 Einfluss des Caspase-Inhibitors Z-VAD-FMK auf die Stabilität des de *novo* synthetisierten Membranproteins Mel-hEGFR-eYFP in einem zellfreien eukaryotischen Translationssystem basierend auf Sf21 Insektenzelllysaten im Batch-Modus

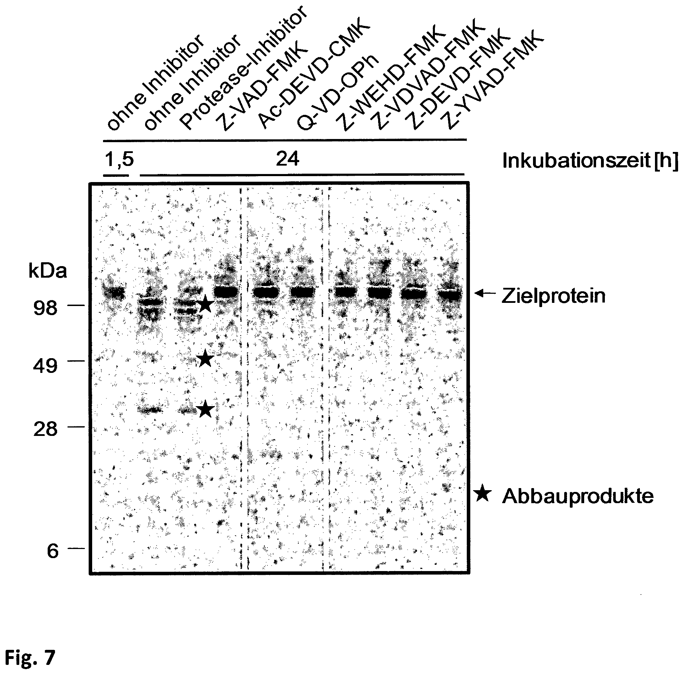

**Fig.** 7 Einfluss verschiedener Caspase-Inhibitoren (Z-VAD-FMK; Ac-DEVD-CMK; Q-VD-OPh, Z-WEHD-FMK, Z-VDVAD-FMK, Z-DEVD-FMK, Z-YVAD-FMK) auf die Stabilität des de *novo* synthetisierten Membranproteins Mel-hEGFR-eYFP in einem zellfreien eukaryotischen Translationssystem basierend auf Sf21 Insektenzelllysaten im Batch-Modus

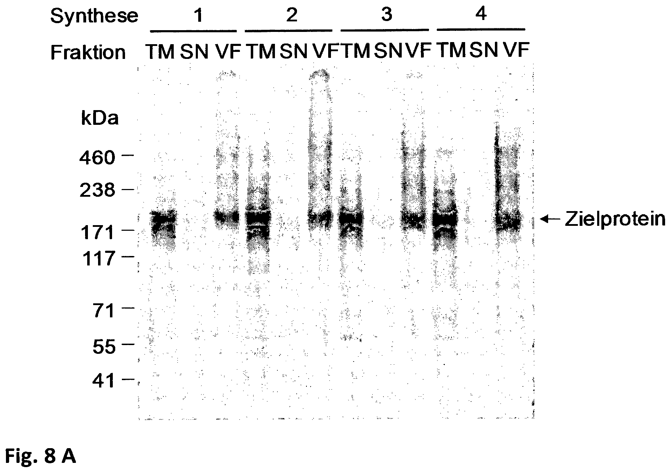

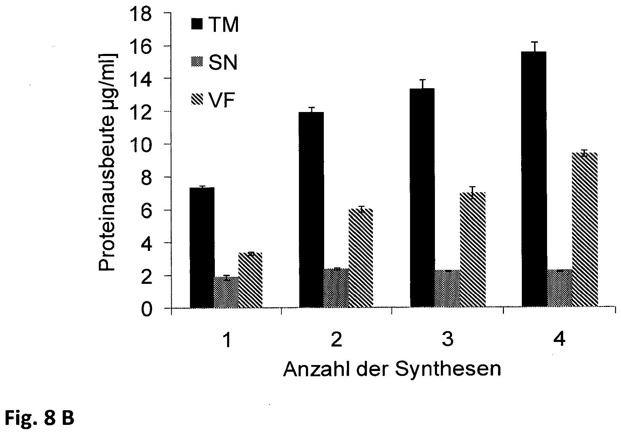

**Fig.** 8 Repetitive Synthesen des Membranproteins Mel-hEGFR-eYFP in die Mikrosomen des Insektenzelllysates in Gegenwart des Caspase-Inhibitors Z-VAD-FMK

Die folgenden Beispiele dienen zur näheren Erläuterung der Erfindung, ohne diese jedoch auf die speziellen Rahmenbedingungen und Parameter dieser Beispiele zu beschränken.

BEISPIEL 1

*Einfluss des Caspase-Inhibitors auf die Expression eines zytosolischen Proteins in einem zellfreien Translationssystem unter Verwendung eines eukaryotischen Zellextraktes* aus *Spodoptera frugiperda*

Der Einfluss des Caspase-Inhibitors auf die maximal erzielbare Proteinausbeute wurde zunächst anhand der Expression des zytosolischen Proteins SII-eYFP untersucht. Bei diesem Modellprotein handelt es sich um das *enhanced yellow fluorescent protein (eYFP)* (= gelb fluoreszierendes Protein), welches am N-Terminus mit einem Affinitätstag fusioniert wurde (Strep-Tag, SII-Tag) und im Vektor pIX3.0 (Qiagen) vorlag.

Die Expression des Modellproteins wurde im Batch- und im Dialyse-System (50 µl Reaktionskammer; 1000 µl Feeding-Kammer; *Cut-off* der Membran = 10 kDa) über einen Zeitraum von 48 h analysiert. Die Translationsansätze wurden im Batch- und im Dialyse-System jeweils mit (+) und ohne (-) Insektenzellvesikel (V) sowie mit (+) und ohne (-) Caspase-Inhibitor (CI) inkubiert (bei 27°C und 600 rpm). Die Proteinsynthese wurde in Gegenwart der radioaktiv markierten Aminosäure $^{14}$C-Leucin durchgeführt, um die Menge an de *novo* synthetisiertem Zielprotein mittels heißer TCA-Fällung und Szintillationsmessung bestimmen zu können.

Die Translationsansätze wurden zu bestimmten Zeitpunkten (0 h, 2 h, 4 h, 24 h und 48 h) unterbrochen und wie folgt analysiert: Jeweils 5 µl des Ansatzes wurden in heißer TCA bzw. eiskaltem Aceton gefällt. Weitere 5 µl wurden in 25 µl PBS resuspendiert. Das restliche Probenvolumen wurde durch einen Zentrifugationsschritt in die Überstands-(SN) und die vesikuläre Fraktion (VF) separiert. Aliquote dieser Fraktionen (5 µl) wurden jeweils in 25 µl PBS verdünnt.

Nach der TCA-Fällung wurden die Proben über eine Vakuumbetriebene Filtrationsanlage von freien radioaktiven Aminosäuren getrennt und einer Szintillationsmessung unterzogen. Die in Aceton gefällten Proteine wurden nach Trocknung in reduzierendem Probenpuffer aufgenommen und elektrophoretisch aufgetrennt.

Die in PBS resuspendierten Proben wurden im Phosphorimager System (Typhoon TRIO+ Imager, GE Healthcare) in Hinblick auf ihre Fluoreszenzintensität untersucht. Dazu wurden 25 µl Probe je Kavität des Ibidi-Slides pipettiert und vermessen, wobei die Anregung der Proben bei 488 nm erfolgte und die Emission bei 526 nm vermessen wurde.

Die in Fig. 1 dargestellten Resultate zeigen, dass ohne den Zusatz des Caspase-Inhibitors eine moderate Steigerung der Proteinmenge (ca. 50%) im Dialyse-System im Vergleich zum Batch-System erzielt wurde (Batch-System nach 48 h, - V, - CI = 17,5 µg/ml; Dialyse-System nach 48 h, - V, - CI = 27,1 µg/ml). Im Gegensatz dazu führte die Zugabe des Caspase-Inhibitors zum Translationsansatz zu einer drastischen Steigerung (ca. 400%) der erzielten, maximalen Proteinausbeute von 17,3 µg/ml (Batch-System nach 48 h, - V, + CI) auf 90,8 µg/ml Zielprotein (Dialyse-System nach 48 h, - V, + CI) (Fig. 1A). Die Fluoreszenzaufnahmen der Proben zeigten eine ähnliche Tendenz. Das detektierte Fluoreszenzsignal der Translationsansätze, die im Dialyse-System in Gegenwart des Caspase-Inhibitors synthetisiert wurden, unterschieden sich deutlich von den übrigen Proben (Fig. 1C). Die densitometrische Auswertung dieser Proben offenbart ein 3,7-fach intensiveres Fluoreszenzsignal der im Dialyse-System - CI (nach 48 h, - V) exprimierten Proben im Vergleich zum Dialyse-System + CI (nach 48 h, - V) (Fig. 1D).

BEISPIEL 2

*Einfluss des Caspase-Inhibitors auf die Expression von Membranproteinen in einem zellfreien Translationssystem unter Verwendung eines eukaryotischen Zellextraktes aus Spodoptera frugiperda*

Hier wurde der Einfluss des Caspase-Inhibitors auf die Expression verschiedener Membranproteine im eukaryotischen Translationssystem näher untersucht. Zu diesem Zweck wurden drei verschiedene Modellproteine ausgewählt: Beim Endothelin-B Rezeptor (ETB) handelt es sich um einen G-Protein gekoppelten Rezeptor mit sieben Transmembrandomänen, der kloniert in den Vektor pIX3.0 (Qiagen) für die Expression eingesetzt wurde. Weiterhin wurde das Typ-I Transmembranprotein *Heparinbinding EGF-like growth factor* (Hb-EGF), N-terminal fusioniert an eine Melittin-Signalsequenz und C-terminal fusioniert an eYFP, vorliegend im Vektor pIX3.0, für die Expression verwendet. Zusätzlich wurde die Expression des Membranproteins Bakteriorhodopsin untersucht, welches ebenfalls sieben Transmembrandomäne besitzt und im Vektor pMA (Geneart) vorlag.

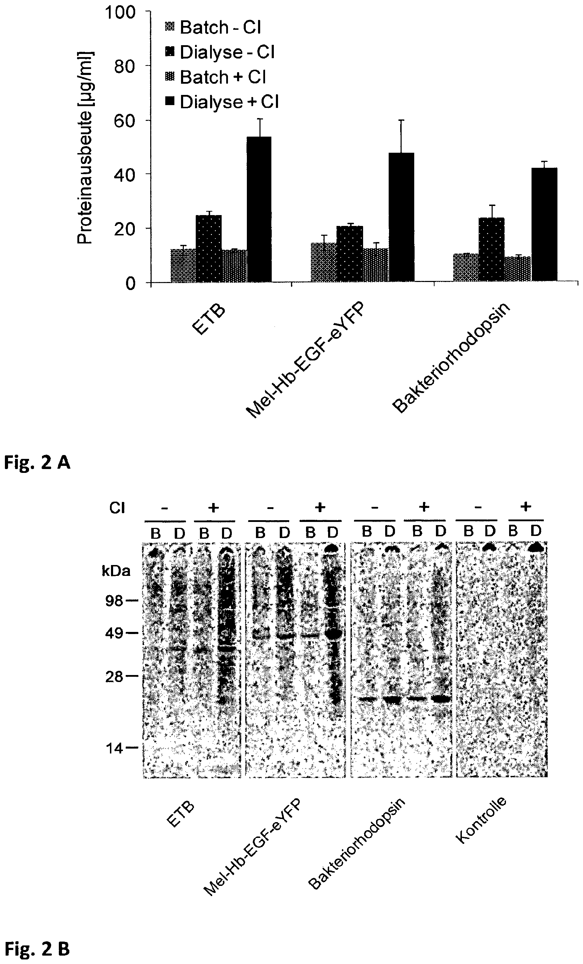

Die drei Proteine wurden jeweils im Batch- und im Dialyse-System ohne (-) und mit (+) Zusatz des Caspase-Inhibitors (CI) in Gegenwart von $^{14}$C-Leucin für 48 h exprimiert (27°C; 600 rpm, Eppendorf Thermomixer Comfort) und wie in Beispiel 1 analysiert. Die Bestimmung der erzielten Proteinausbeuten mittels Szintillationsmessung zeigt einen deutlichen Einfluss des Caspase-Inhibitors auf die maximal erzielbaren Proteinausbeuten. Die Expression jedes der drei Membranproteine (ETB, 49,3 kDA; Hb-EGF, 51 kDA; Bakteriorhodopsin, 26,9 kDA) konnte im Dialyse-System (Vergleich der Dialyse-Ansätze + CI und - CI) unter Zusatz des Caspase-Inhibitors um ca. 100% gesteigert werden (Fig.2).

BEISPIEL 3

*Einfluss des reduzierenden Agens Dithiothreitol (DTT) auf die maximal erzielbaren Proteinausbeuten unter Verwendung eines eukaryotischen Zellextrakts und Zusatz des Caspase-Inhibitors*

Der Zusatz reduzierender Agenzien wie DTT zu Zellextrakten und Translationspuffern diente bisher der Verlängerung der Lagerungsfähigkeit. Untersuchungen zur zellfreien Expression von Proteinen mit Disulfidbrücken haben jedoch ergeben, dass reduzierende Agenzien eine Bildung von Disulfidbrücken im Zielprotein inhibieren können (Katzen, F., G. Chang, and W. Kudlicki, Trends Biotechnol., 2005. 23(3): S. 150-156). Nachdem Disulfidbrücken bei vielen Proteinen wichtige posttranslationale Modifikationen darstellen, die dem Protein Stabilität geben und einen wesentlichen Beitrag zur Proteinfaltung leisten, wurden verschiedene Versuche unternommen, Translationssysteme mit einem definierten Redoxpotential zu entwickeln. Zu diesem Zweck wird in den meisten Fällen auf den Zusatz reduzierender Agentien zum Lysat oder dem Translationspuffer verzichtet.

Das hier beschriebene zellfreie Translationssystem soll eine Plattform bieten, mit welcher komplexe eukaryotische Proteine synthetisiert werden können. Es war daher von besonderem Interesse festzustellen, ob das erfindungsgemäße Verfahren auch vorteilhaft zur Synthese von Proteinen mit Disulfidbrücken eingesetzt werden kann, und Optimierungsmöglichkeiten aufzuzeigen.

Dazu wurde der Einfluss des reduzierenden Agenz DTT auf die Syntheseleistung des Batch- und Dialyse-Systems bei verschiedenen Modellproteinen untersucht.

Die Synthese des Glykoproteins Erythropoietin (N-terminal fusioniert an eine Melittin-Signalsequenz; Mel-EPO; 20,9 kDa, unglykosyliert) sowie von ETB (49,3 kDa), Luciferase (60,6 kDa), Mel-Hb-EGF-eYFP (51 kDa) und Bakteriorhodopsin (26,9 kDa) erfolgte im Batch (B)- und Dialyse-System (D) unter Zusatz des Caspase-Inhibitors (Z-VAD-FMK) ohne (-) und mit (+) Zusatz von DTT im Translationspuffer in Gegenwart von $^{14}$C-Leucin für 48 h, 27°C und 600 rpm.

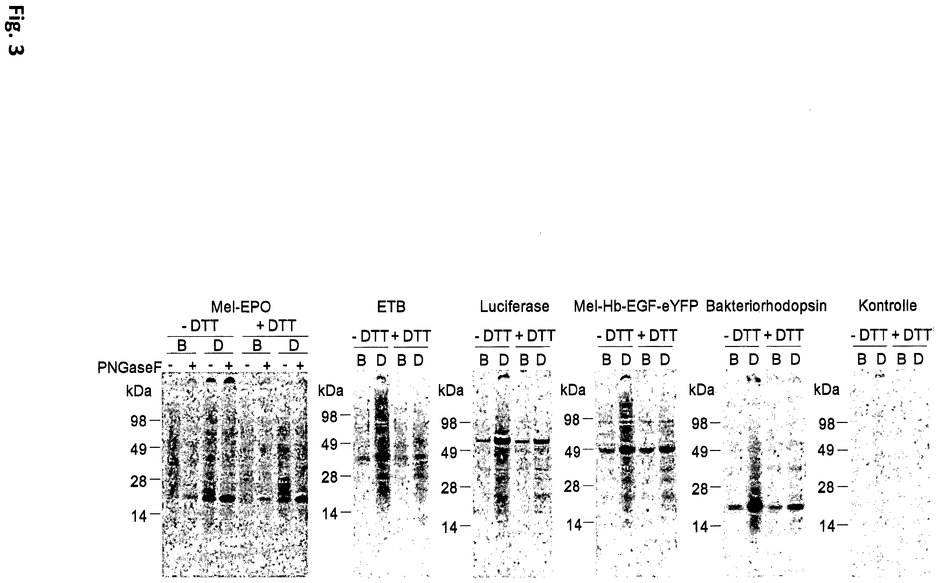

Fig. 3 zeigt das Autoradiogramm der im Batch- und Dialyse-System synthetisierten Proteine. Kontrolle = Translationsansatz ohne Zugabe einer DNA-Matrize.

Es zeigte sich, dass die Abwesenheit von DTT keinen negativen Effekt auf die Expression verschiedener Modellproteine ausübt, darunter zytosolische Proteine (Luciferase, SII-eYFP; beide vorliegend im Expressionsvektor pIX3.0, Qiagen) sowie Membranproteine (ETB, Mel-Hb-EGF-eYFP, Bakteriorhodopsin).

Im Falle des glykosylierten Proteins Erythropoietin (N-terminal fusioniert an eine Melittin-Signalsequenz; Mel-EPO) konnte jedoch gezeigt werden, dass nur in Anwesenheit von DTT eine vollständige Glykosylierung des Zielproteins erzielt werden konnte.

BEISPIEL 4

*Untersuchung der Expression des Typ-I-Transmembranproteins Mel-Hb-EGF-eYFP unter Verwendung eines eukaryotischen Zellextrakts und Zusatz des Caspase-Inhibitors*

Aus den obigen Daten ergibt sich, dass der Zusatz des Caspase-Inhibitors zur Proteinsynthesereaktion die erzielbaren Maximalausbeuten an Membranprotein im Dialyse-System um durchschnittlich ca. 100% steigern kann. Für das Transmembranprotein Mel-Hb-EGF-eYFP konnte eine Zunahme der Gesamtproteinmenge von 20,5 $\mu$g/ml (Dialyse 48 h - CI) auf 47,6 $\mu$g/ml (Dialyse 48 h + CI) verzeichnet werden (= 130%) . Beim Vergleich des Batch-Systems mit dem Dialyse-System konnte eine Zunahme der Gesamtproteinmenge von 12,1 $\mu$g/ml (Batch 48 h + CI) auf 47,6 $\mu$g/ml (Dialyse 48 h + CI) verzeichnet werden (= 300%) (Fig. 2).

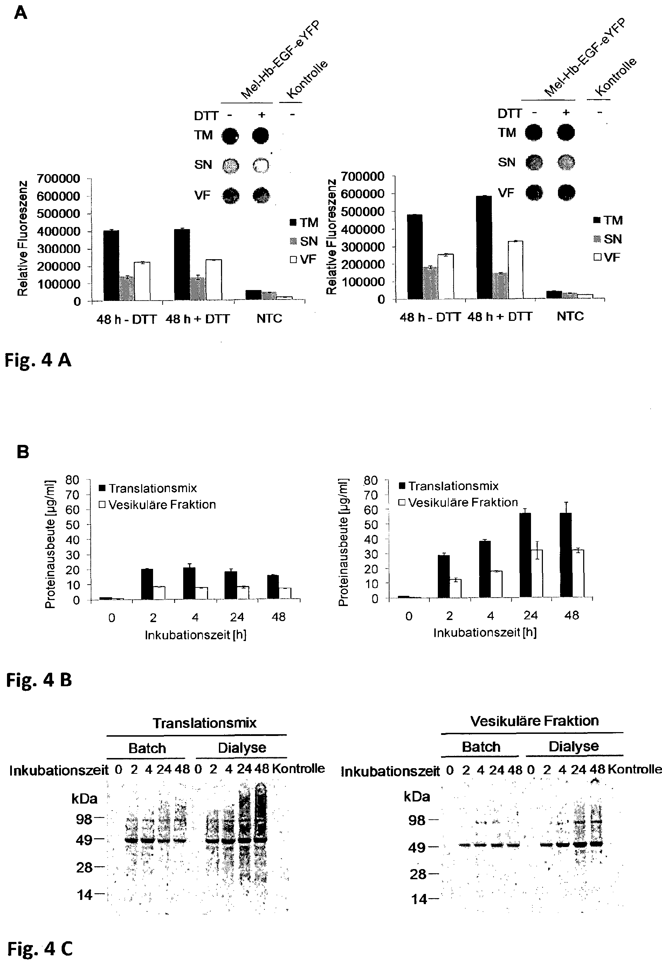

Hier sollte nun untersucht werden, ob die Erhöhung der Gesamtproteinmenge auch mit einer Erhöhung der Menge an transloziertem und in die Lipidschicht der Vesikel integriertem Membranprotein einhergeht. Zu diesem Zweck wurden die Translationsansätze von Mel-Hb-EGF-eYFP durch einen Zentrifugationsschritt in die Überstands- (SN) und die vesikuläre Fraktion (VF) separiert. Aliquote (5 $\mu$l) dieser Proben dienten zur Bestimmung der Menge an de *novo* synthetisiertem Protein oder wurden im Hinblick auf ihre Fluoreszenzeigenschaften untersucht (Fig. 4).

Fig. 4A: Die Synthese von Mel-Hb-EGF-eYFP erfolgte im Batch (links)- und Dialyse-System (rechts) in Abwesenheit (-) und in Gegenwart (+) von DTT unter Zusatz des Caspase-Inhibitors (Z-VAD-FMK) über 48 h bei 27°C und 600 rpm. Nach der Synthese wurden die Ansätze durch einen Zentrifugationsschritt in die Überstands- (SN) und die vesikuläre Fraktion (VF) separiert.

Zur Analyse der Fluoreszenzintensität der Proben wurden Aliquote der Fraktionen (5 $\mu$l) in jeweils 25 $\mu$l PBS verdünnt und 25 $\mu$l dieser Mischung je Kavität des Ibidi-Slides pipettiert und vermessen. Die Anregung der Proben erfolgte bei 488 nm am Phosphorimager (Typhoon TRIO+ Imager, GE Healthcare) und die Emission wurde bei 526 nm vermessen.

Fig. 4B zeigt eine grafische Darstellung der über den Einbau von $^{14}$C-Leucin ermittelten Proteinausbeuten von Mel-Hb-EGF-eYFP im Translationsmix (TM) und der vesikulären Fraktion (VF) über einen Zeitraum von 48 h analysiert im Batch-(links) und Dialyse-System (rechts).

Fig. 4C zeigt ein Autoradiogramm zur Darstellung von $^{14}$C-Leucin markiertem Mel-Hb-EGF-eYFP. Das Protein weist eine apparente molekulare Masse von ca. 51 kDa auf.

Diese Daten belegen, dass die Anwendung des Dialyse-Systems unter Zusatz des Caspase-Inhibitors und in Abwesenheit von DTT zu einer deutlichen Steigerung des Anteils an Mel-Hb-EGF-eYFP in der vesikulären Fraktion des Lysats führt (Batch 48 h - DTT + CI = 6,5 $\mu$g/ml; Dialyse 48 h - DTT + CI = 31,4 pg/ml). Weiterhin zeigt die zeitabhängige Analyse von Proben der vesikulären Fraktion im Dialyse-System eine kontinuierliche Steigerung der Proteinmenge über 24 h, wohingegen im Batch-System das Maximum nach 2 h erreicht ist (Fig. 4B, 4C).

BEISPIEL 5

*Einfluss irreversibler und reversibler Caspase-Inhibitoren auf die Syntheseleistung im eukaryotischen Translationssystem auf Basis eines Zellextraktes aus Spodoptera frugiperda (Sf21) Insektenzellen*

Am Beispiel des fluoreszierenden Proteins SII-eYFP wurde der Einfluss verschiedener irreversibler Caspase-Inhibitoren (Z-VAD-FMK, Ac-VAD-CMK, Ac-DEVD-CMK, Q-VD-OPh) und eines reversiblen Caspase-Inhibitors (Ac-AAVALLPAVLLALLAPDEVD-CHO) auf die Syntheseleistung im eukaryotischen Dialyse-Translationssystem untersucht.

Die Translation von SII-eYFP erfolgte unter Verwendung des DNA-Templates pIX3.0-SII-eYFP über 48 h bei 600 rpm und 27°C im Batch-und Dialyse-Modus in Gegenwart verschiedener irreversibler und reversibler Caspase-Inhibitoren. Alle Inhibitoren wurden in einer Konzentration von 30 $\mu$M eingesetzt. Nach erfolgter Translation wurden die verschiedenen Ansätze im Phosphorimager System (Typhoon TRIO+ Imager, GE Healthcare) in Hinblick auf ihre Fluoreszenzintensität untersucht. Zu diesem Zweck wurden 5 $\mu$l jedes Translationsansatzes in 25 $\mu$l PBS resuspendiert. Anschließend wurden 25 $\mu$l dieser Probe je Kavität eines Ibidi-Slides pipettiert und vermessen, wobei die Anregung der Proben bei 488 nm erfolgte und die Emission bei 526 nm vermessen wurde.

Die in Fig. 5 dargestellten Ergebnisse zeigen, dass alle getesteten irreversiblen Caspase-Inhibitoren die Syntheseleistung um minimal 77% (Q-VD-OPh) bis zu maximal 122% (Ac-DEVD-CMK) steigern können im Vergleich zu einem Kontroll-Dialyse-Ansatz ohne Inhibitor. Der getestete reversible CaspaseInhibitor Ac-AAVALLPAVLLALLAPDEVD-CHO bewirkte eine Steigerung der Ausbeute an fluoreszierendem Protein um 53% im Vergleich zum Dialyse-Ansatz ohne Inhibitor.

Anhand der beschriebenen Daten kann demnach geschlussfolgert werden, dass prinzipiell sowohl mit irreversiblen als auch reversiblen Inhibitoren eine Steigerung der Syntheseleistung im eukaryotischen Dialyse-System möglich ist.

BEISPIEL 6

*Einfluss des Caspase-Inhibitors Z-VAD-FMK auf die Stabilität eines de novo synthetisierten Membranproteins im eukaryotischen Translationssystem*

Der positive Einfluss des Caspase-Inhibitors auf die Stabilität zellfrei synthetisierter Proteine wird hier anhand der Expression des EGF-Rezeptors aufgezeigt, wobei es sich um ein humanes, hochmolekulares Transmembranprotein *(Epidermal Growth Factor Receptor)* mit intrinsischer TyrosinkinaseAktivität handelt. Die kodierende Sequenz des EGF-Rezeptors wurde N-terminal mit einer Melittin-Signalsequenz (Mel) und C-terminal mit dem gelb-fluoreszierenden Protein (eYFP) fusioniert und in den Vektor pIX3.0 (Qiagen) kloniert. Im Folgenden wird das Modellprotein als Mel-hEGFR-eYFP (= 163 kDa) bezeichnet. Die Melittin-Signalsequenz ermöglicht eine Translokation des Membranproteins in die Mikrosomen des eukaryotischen Zellextraktes und in der Folge eine Einbettung in die Membran der Mikrosomen. Mel-hEGFR-eYFP besitzt neun potentielle N-Glycosylierungsstellen, daher wird durch die Translokation des Zielproteins auch eine N-Glykosylierung des Zielproteins im zellfreien System ermöglicht.

Fig. 6 zeigt die zellfreie Expression von Mel-hEGFR-eYFP in einem Batch-basierten eukaryotischen Translationssystem basierend auf Sf21-Insektenzelllysaten in Gegenwart von $^{14}$C-Leucin. Die Translationsreaktion wurde in Abwesenheit und in Gegenwart eines Caspase-Inhibitors (Z-VAD-FMK, Promega, 30 $\mu$M) bzw. eines Protease-Inhibitor-Mixes ("Complete Protease Inhibitor Cocktail", Roche) durchgeführt und zu den angegebenen Inkubationszeiten (1,5 h, 5 h und 24 h) gestoppt durch Einfrieren der Translationsreaktion in flüssigem Stickstoff. Die Analyse der de *novo* synthetisierten Zielproteine erfolgte mittels SDS-PAGE und Autoradiographie. Zur Durchführung der SDS-PAGE wurden 5 $\mu$l des Translationsansatzes in eiskaltem Aceton gefällt. Die Proteinpellets wurden nach ihrer Trocknung in reduzierendem Probenpuffer aufgenommen und in einer 10 %-igen SDS-PAGE aufgetrennt. Die Visualisierung der zellfrei synthetisierten und $^{14}$C-Leucin-markierten Proteine erfolgte unter Anwendung des Phosphorimager Systems (Typhoon TRIO+ Imager, GE Healthcare).

Das Autoradiogramm zeigt zwei distinkte Banden des Zielproteins nach 1,5 h Inkubationszeit. Aufgrund der zahlreichen potentiellen N-Glykosylierungsstellen in der Sequenz des Zielproteins wird vermutet, dass es sich bei der Bande mit dem größeren Molekulargewicht um das Protein mit einer oder mehreren angefügten N-Glykosylierungen handelt. Die Proteinbande mit dem geringeren Molekulargewicht entspräche demzufolge dem Zielprotein ohne Zuckerreste. Ohne Zusatz des Caspase-Inhibitors lässt sich für Mel-hEGFR-eYFP bereits nach 5 h Inkubationszeit feststellen, dass die Bandenintensität des Zielproteins im Autoradiogramm abnimmt und niedermolekulare Abbauprodukte sichtbar werden. Im Gegensatz dazu zeigen die Proteine, die in Gegenwart des Caspase-Inhibitors synthetisiert wurden, verminderte Anzeichen eines proteolytischen Abbaus. Auch nach 24 h Inkubationszeit lässt sich das Membranprotein Mel-hEGFR-eYFP in intakter Form im Autoradiogramm nachweisen. Der spezifische Effekt des Caspase-Inhibitors wird dadurch deutlich, dass der Zusatz eines kommerziell erhältlichen Protease-Inhibitor-Gemisches ("Complete Protease Inhibitor Cocktail", Roche) nicht die stabilisierende Wirkung des Caspase-Inhibitors erzielte.

BEISPIEL 7

*Positiver Einfluss verschiedener Caspase-Inhibitor-Typen auf die Stabilität eines de novo synthetisierten Membranproteins im eukaryotischen Translationssystem*

Im folgenden Versuch wird die Wirkung verschiedener Caspase-Inhibitoren (Z-VAD-FMK, Promega; Ac-DEVD-CMK, SantaCruz Biotechnology; Q-VD-OPh, Z-WEHD-FMK, Z-VDVAD-FMK, Z-DEVD-FMK, Z-YVAD-FMK, R&D Systems; alle 30 $\mu$M) mit unterschiedlichen funktionellen Gruppen (Methylketon, Fluormethyketon, FMK oder Chlormethylketon, CMK) und Peptidresten auf die Synthese des Zielproteins Mel-hEGFR-eYFP in einem zellfreien eukaryotischen Translationssystem basierend auf Sf21 Insektenzelllysaten im Batch-Modus in Gegenwart von $^{14}$C-Leucin untersucht. Die Synthese wurde zu den in Fig. 7 angegebenen Inkubationszeiten (1,5 h; 24 h) gestoppt.

Die vorliegenden Ergebnisse zeigen, dass alle eingesetzten Inhibitoren die Stabilität des Zielproteins im Translationsansatz auch nach langen Inkubationszeiten (24 h) gewährleisten (Fig. 7).

BEISPIEL 8

*Anwendung des Caspase-Inhibitors Z-VAD-FMK bei Mehrfach-Synthese eines Zielproteins in mikrosomalen Vesikeln*

Eine geeignete Methode, die Konzentration eines bestimmten Zielproteins im Lumen oder der Membran der Insektenzellvesikel zu erhöhen, besteht in der Durchführung von Mehrfach-Synthesen. Diese Vorgehensweise führt zu einer längeren Verweilzeit der de *novo* synthetisierten Zielproteine im Translationsansatz. Zur erfolgreichen Umsetzung ist es daher zwingend erforderlich, die Stabilität der Zielproteine auch nach längeren Inkubationszeiten (> 1,5 h) im Translationsansatz zu gewährleisten. Zu diesem Zweck wurde der Translationsreaktion der Caspase-Inhibitor Z-VAD-FMK zugesetzt. Die Mehrfach-Synthese des Zielproteins wurde wie folgt durchgeführt: Die Mikrosomen des unter Standardbedingungen inkubierten Translationsansatzes (27 °C, 1,5 h) wurden durch einen Zentrifugationsschritt bei 16.000 g pelletiert und in einem neuen translationsaktiven Zelllysat ohne Mikrosomen resuspendiert und erneut 1,5 h bei 27 °C in Gegenwart des Caspase-Inhibiors Z-VAD-FMK inkubiert. Im vorliegenden Versuchsbeispiel wurde die Synthese vier Mal wiederholt. Nach jedem Syntheseschritt wurde die Ausbeute an radioaktiv markiertem Protein im Translationsmix, dem Überstand und der vesikulären Fraktion bestimmt. Zusätzlich wurden 5 μl des Translationsmixes in Aceton gefällt und anschließend elektrophoretisch aufgetrennt. Das dazugehörige Autoradiogramm (Fig. 8A) zeigt die Proteinbanden von Mel-hEGFR-eYFP im Translationsmix und der vesikulären Fraktion des Translationsansatzes. Die Zugabe des Caspase-Inhibitors zur Translationsreaktion ermöglichte eine Synthese des Zielproteins über insgesamt 6 h.

Die vorliegenden Daten zeigen, dass die Ausbeute an Zielprotein von Syntheseschritt 1 bis 4 um 110 % (Gesamtprotein im Translationsmix) bzw. um 180 % (Vesikuläre Fraktion) gesteigert werden konnten.

## Patentansprüche

1. Verfahren zur zellfreien Proteinsynthese, welches eine *in* vitro-Translationsreaktion unter Verwendung einer Nukleinsäure-Matrize und eines eukaryotischen Zelllysats umfasst, **dadurch gekennzeichnet, dass** die Translationsreaktion in Gegenwart eines Caspase-Inhibitors durchgeführt wird und das Verfahren in einer Vorrichtung durchgeführt wird, die mindestens zwei durch eine Dialysemembran getrennte Kompartimente umfasst, wobei die Translationsreaktion in mindestens einem ersten Kompartiment, dem Reaktionskompartiment, stattfindet und während der Translationsreaktion durch die Dialysemembran i) Reaktanden aus mindestens einem weiterem Kompartiment, dem Ver- und Entsorgungskompartiment, in das Reaktionskompartiment diffundieren und ii) Reaktions-nebenprodukte aus dem Reaktionskompartiment in das Ver- und Entsorgungskompartiment diffundieren.

2. Verfahren nach Anspruch 1, **dadurch gekennzeichnet, dass** der Caspase-Inhibitor in einer Konzentration von 20 $\mu$M bis 100 $\mu$M, vorzugsweise 25 bis 50 $\mu$M, in der Reaktionsmischung vorliegt.

3. Verfahren nach einem der Ansprüche 1 bis 2, **dadurch gekennzeichnet, dass** das eukaryotische Zelllysat aus der Gruppe, die Weizenkeimlysate, Insektenzelllysate, insbesondere Sf21-Zelllysate, Retikulozytenlysate, Keratinocytenlysate, Zellextrakte aus CHO-Zellen, HeLa-Zellen, Hybridomzellen oder kultivierten Lymphomzellen, umfasst, ausgewählt ist.

**4.** Verfahren nach einem der Ansprüche 1 bis 3, **dadurch gekennzeichnet, dass** das Zelllysat Membranvesikel enthält.

**5.** Verfahren nach Anspruch 4, **dadurch gekennzeichnet, dass** die Membranvesikel aus derselben Zelllinie wie das Zelllysat stammen.

**6.** Verfahren nach einem der Ansprüche 1 bis 5, **dadurch gekennzeichnet, dass** eine maximale Proteinausbeute von mindestens 30 $\mu$g/ml, vorzugsweise mindestens 50 $\mu$g/ml, 100 $\mu$g/ml oder 150 $\mu$g/ml, erzielt wird.

**7.** Verwendung eines Caspase-Inhibitors zu Steigerung der Proteinausbeute in einem zellfreien kontinuierlichen Verfahren zur Proteinsynthese unter Verwendung eines eukaryotischen Zelllysats, **dadurch gekennzeichnet, dass** das Verfahren in einer Vorrichtung durchgeführt wird, die mindestens zwei durch eine Dialysemembran getrennte Kompartimente umfasst, wobei die Translationsreaktion in mindestens einem ersten Kompartiment, dem Reaktionskompartiment, stattfindet und während der Translationsreaktion durch die Dialysemembran i) Reaktanden aus mindestens einem weiterem Kompartiment, dem Ver- und Entsorgungskompartiment, in das Reaktionskompartiment diffundieren und ii) Reaktions-nebenprodukte aus dem Reaktionskompartiment in das Ver- und Entsorgungskompartiment diffundieren.

**8.** Verwendung nach Anspruch 7, **dadurch gekennzeichnet, dass** die maximale Proteinausbeute, gemessen in $\mu$g/ml Reaktionsmedium, um einen Faktor von mindestens 2, insbesondere mindestens 5 oder 10, gesteigert wird.

**9.** Verfahren nach einem der Ansprüche 1 bis 6 oder Verwendung nach einem der Ansprüche 7 oder 8, **dadurch gekennzeichnet, dass** der Caspase-Inhibitor ein Aminosäure- oder Peptidderivat ist, umfassend eine Aminosäure oder Peptidsequenz, die als Substrat für eine Caspase, insbesondere eine oder mehrere der Caspase-Typen 1-14, dient, sowie eine funktionelle Gruppe, welche irreversibel oder reversibel an eine Caspase, insbesondere eine oder mehrere der Caspase-Typen 1-14, bindet.

**10.** Verfahren oder Verwendung nach Anspruch 9, **dadurch gekennzeichnet, dass** der Caspase-Inhibitor die Aminosäure Aspartat oder eine Peptidsequenz, welche die Aminosäure Aspartat enthält, umfasst.

**11.** Verfahren oder Verwendung nach Anspruch 10, **dadurch gekennzeichnet, dass** die Aminosäure oder Peptidsequenz aus der Gruppe aus Aspartat, Valin-Alanin-Aspartat (VAD), Aspartat-Glutamat-Valin-Aspartat (DEVD) und Tyrosin-Valin-Alanin-Aspartat (YVAD) ausgewählt ist.

**12.** Verfahren nach einem der Ansprüche 1 bis 6 oder Verwendung nach einem der Ansprüche 7 oder 8, **dadurch gekennzeichnet, dass** der Caspase-Inhibitor ein irreversibler Inhibitor ist, welcher als funktionelle Gruppe eine Methylketongruppe umfasst.

**13.** Verfahren oder Verwendung nach Anspruch 12, **dadurch gekennzeichnet, dass** der Caspaseinhibitor eine Fluormethylketon- (FMK), Chlormethylketon- (CMK) oder Difluorphenoxmethylketongruppe, welche irreversibel an alle Caspase-Typen 1-14 bindet, umfasst.

**14.** Verfahren nach einem der Ansprüche 1 bis 6 oder Verwendung nach einem der Ansprüche 7 oder 8, **dadurch gekennzeichnet, dass** der Caspase-Inhibitor ein an mindestens eine Aldehydgruppe gekoppeltes Peptid darstellt.

**15.** Verwendung nach Anspruch 7 oder 8, **dadurch gekennzeichnet, dass** der Caspase-Inhibitor in einer Konzentration von 20 $\mu$M bis 100 $\mu$M, vorzugsweise 25 bis 50 $\mu$M, besonders bevorzugt etwa 30 $\mu$M, in der Reaktionsmischung vorliegt.

**Claims**

**1.** A method for cell-free protein synthesis which comprises an *in vitro* translation reaction using a nucleic acid template and a eukaryotic cell lysate, **characterised in that** the translation reaction is carried out in the presence of a caspase inhibitor and the method is carried out in a device which comprises at least two compartments separated by a dialysis membrane, wherein the translation reaction takes place in at least one first compartment, the reaction compartment, and, during the translation reaction, i) reactants diffuse through the dialysis membrane out of at least one further compartment, the supply and discharge compartment, into the reaction compartment and ii) reaction by-products diffuse through the dialysis membrane out of the reaction compartment into the supply and discharge compartment.

**2.** The method according to claim 1, **characterised in that** the caspase inhibitor is present in a concentration of 20 $\mu$M to 100 $\mu$M, preferably 25 $\mu$M to 50 $\mu$M, in the reaction mixture.

**3.** The method according to one of the claims 1 to 2, **characterised in that** the eukaryotic cell lysate is selected from the group comprising wheatgerm lysates, insect cell lysates, in particular *Sf21* cell lysates, reticulocyte lysates, keratinocyte lysates, cell extracts from CHO cells, HeLa cells, hybridoma cells or cultivated lymphoma cells.

**4.** The method according to one of the claims 1 to 3, **characterised in that** the cell lysate contains membrane vesicles.

**5.** The method according to claim 4, **characterised in that** the membrane vesicles originate from the same cell line as the cell lysate.

**6.** The method according to one of the claims 1 to 5, **characterised in that** a maximum protein yield of at least 30 $\mu$g/ml,preferably at least 50 $\mu$g/ml,100 $\mu$g/ml or 150 $\mu$g/ml,is achieved.

**7.** Use of a caspase inhibitor to increase the protein yield in a cell-free continuous process for protein synthesis using a eukaryotic cell lysate, **characterized in that** the process is carried out in a device which comprises at least two compartments separated by a dialysis membrane, wherein the translation reaction takes place in at least one first compartment, the reaction compartment, and, during the translation reaction, i) reactants diffuse through the dialysis membrane out of at least one further compartment, the supply and discharge compartment, into the reaction compartment and ii) reaction by-products diffuse through the dialysis membrane out of the reaction compartment into the supply and discharge compartment.

**8.** The use according to claim 7, **characterised in that** the maximum protein yield, measured in $\mu$g/ml of reaction medium, is increased by a factor of at least 2, particularly at least 5 or 10.

**9.** The method according to one of the claims 1 to 6 or the use according to one of claims 7 or 8, **characterised in that** the caspase inhibitor is an amino acid derivative or a peptide derivative comprising an amino acid or peptide sequence which serves as a substrate for a caspase, in particular one or more of the caspase types 1-14, and a functional group which irreversibly or reversibly binds to a caspase, in particular to one or more of the caspase types 1-14.

**10.** The method or use according to claim 9, **characterised in that** the caspase inhibitor comprises the amino acid aspartate or a peptide sequence, which contains the amino acid aspartate.

**11.** The method or use according to claim 10, **characterised in that** the amino acid or peptide sequence is selected from the group consisting of aspartate, valine-alanine-aspartate (VAD), aspartate-glutamate-valine-aspartate (DEVD) and tyrosine-valine-alanine-aspartate (YVAD).

**12.** The method according to one of the claims 1 to 6 or use according to one of the claims 7 or 8, **characterised in that** the caspase inhibitor is an irreversible inhibitor which comprises a methylketone group as a functional group.

**13.** The method or use according to claim 12, **characterised in that** the caspase inhibitor comprises a fluoromethylketone (FMK), chloromethylketone (CMK) or difluorophenoxymethylketone group, which binds irreversibly to all the caspase types 1-14.

**14.** The method according to one of the claims 1 to 6 or use according to one of the claims 7 or 8, **characterised in that** the caspase inhibitor represents a peptide coupled to at least one aldehyde group.

**15.** The use according to claim 7 or 8, **characterised in that** the caspase inhibitor is present in a concentration of 20 $\mu$M to 100 $\mu$M, preferably 25 $\mu$M to 50 $\mu$M, especially preferred about 30 $\mu$M, in the reaction mixture.

**Revendications**

**1.** Procédé de synthèse de protéines acellulaires, lequel comprend une réaction de traduction *in vitro* en utilisant une matrice d'acide nucléique et un lysat cellulaire eucaryote, **caractérisé en ce que** la réaction de traduction est effectuée en présence d'un inhibiteur de la caspase et le procédé est effectué dans un dispositif qui comprend au moins deux compartiments séparés par une membrane de dialyse, dans lequel la réaction de traduction a lieu dans au moins un premier compartiment, le compartiment de réaction, et, pendant la réaction de traduction, i) des réactifs provenant d'au moins un autre compartiment, le compartiment d'alimentation et d'élimination, se diffusent par la membrane de dialyse dans le compartiment de réaction et ii) des sous-produits de réaction provenant du compartiment de réaction se diffusent par la membrane de dialyse dans le compartiment d'alimentation et d'élimination.

**2.** Procédé selon la revendication 1, **caractérisé en ce que** l'inhibiteur de la caspase est présent dans le mélange réactionnel en une concentration de 20 µM à 100 µM, de préférence de 25 à 50 µM.

**3.** Procédé selon l'une quelconque des revendications 1 à 2, **caractérisé en ce que** le lysat cellulaire eucaryote est choisi parmi le groupe, qui comprend des lysats de germes de blé, des lysats cellulaires d'insecte, en particulier des lysats cellulaires Sf21, des lysats de réticulocytes, des lysats de kératinocytes, des extraits cellulaires issus de cellules CHO, des cellules HeLa, des cellules d'hybridomes ou des cellules cultivées de lymphome.

**4.** Procédé selon l'une quelconque des revendications 1 à 3, **caractérisé en ce que** le lysat cellulaire contient des vésicules membranaires.

**5.** Procédé selon la revendication 4, **caractérisé en ce que** les vésicules membranaires proviennent de la même lignée cellulaire que le lysat cellulaire.

**6.** Procédé selon l'une quelconque des revendications 1 à 5, **caractérisé en ce qu'**une production maximale de protéines d'au moins 30 µg/ml, de préférence d'au moins 50 µg/ml, de 100 µg/ml ou de 150 µg/ml, est obtenue.

**7.** Utilisation d'un inhibiteur de la caspase pour augmenter la production de protéines dans un procédé continu acellulaire pour la synthèse de protéines en utilisant un lysat cellulaire eucaryote, **caractérisée en ce que** le procédé est effectué dans un dispositif qui comprend au moins deux compartiments séparés par une membrane de dialyse, dans laquelle la réaction de traduction a lieu dans au moins un premier compartiment, le compartiment de réaction et, pendant la réaction de traduction, i) des réactifs provenant d'au moins un autre compartiment, le compartiment d'alimentation et d'élimination, se diffusent par la membrane de dialyse dans le compartiment de réaction et ii) des sous-produits de réaction provenant du compartiment de réaction se diffusent par la membrane de dialyse dans le compartiment d'alimentation et d'élimination.

**8.** Utilisation selon la revendication 7, **caractérisée en ce que** la production maximale de protéines, mesurée en µg/ml de milieu de réaction, est augmentée d'un facteur d'au moins 2, en particulier d'au moins 5 ou 10.

**9.** Procédé selon l'une quelconque des revendications 1 à 6 ou utilisation selon l'une quelconque des revendications 7 ou 8, caractérisé ou **caractérisée en ce que** l'inhibiteur de la caspase est un dérivé d'acide aminé ou un dérivé de peptide, comprenant un acide aminé ou une séquence de peptide, qui fait office de substrat pour une caspase, en particulier un ou plusieurs des types de caspase 1 - 14, ainsi qu'un groupe fonctionnel, lequel se lie de manière irréversible ou réversible sur une caspase, en particulier une ou plusieurs des types de caspase 1 - 14.

**10.** Procédé ou utilisation selon la revendication 9, caractérisé ou **caractérisée en ce que** l'inhibiteur de la caspase comprend l'acide aminé aspartate ou une séquence de peptide, laquelle contient l'acide aminé aspartate.

**11.** Procédé ou utilisation selon la revendication 10, caractérisé ou **caractérisée en ce que** l'acide aminé ou la séquence de peptide est choisi ou choisie parmi le groupe composé d'aspartate, d'aspartate-alanine-valine (VAD), d'aspartate-valineglutamate-aspartate (DEVD) et d'aspartate-alaninevaline-tyrosine (YVAD).

**12.** Procédé selon l'une quelconque des revendications 1 à 6 ou utilisation selon l'une quelconque des revendications 7 ou 8, caractérisé ou **caractérisée en ce que** l'inhibiteur de la caspase est un inhibiteur irréversible, lequel comprend en tant que groupe fonctionnel un groupe de méthylcétone.

**13.** Procédé ou utilisation selon la revendication 12, caractérisé ou **caractérisée en ce que** l'inhibiteur de la caspase comprend un groupe de fluorométhylcétone (FMK), de chlorométhylcétone (CMK) ou de difluorophénoxyméthylcétone, lequel se lie de manière irréversible sur tous les types de caspase 1 - 14.

**14.** Procédé selon l'une quelconque des revendications 1 à 6 ou utilisation selon l'une quelconque des revendications 7 ou 8, caractérisé ou **caractérisée en ce que** l'inhibiteur de la caspase constitue un peptide couplé à au moins un groupe aldéhyde.

15. Utilisation selon la revendication 7 ou 8, **caractérisée en ce que** l'inhibiteur de la caspase est présent dans le mélange réactionnel en une concentration de 20 $\mu$M à 100 $\mu$M, de préférence de 25 à 50 $\mu$M, de manière particulièrement préférée d'environ 30 $\mu$M.

**Fig. 1 A**

**Fig. 1 B**

| | | Inkubationszeit [h] | | | | | V | CI |
|---|---|---|---|---|---|---|---|---|
| | | 0 | 2 | 4 | 24 | 48 | | |
| TM | Batch | | ● | ● | ● | ● | – | – |
| | | | ● | ● | ● | ● | – | + |
| | | | ○ | ○ | ○ | ○ | + | – |
| | | | ○ | ○ | ○ | ○ | + | + |
| | Dialyse | | ● | ● | ● | ● | – | – |
| | | | ● | ● | ● | ● | – | + |
| | | | ● | ● | ● | ● | + | – |
| | | | ● | ● | ● | ● | + | + |
| SN | Dialyse | | ● | ● | ● | ● | – | – |
| | | | ● | ● | ● | ● | – | + |
| | | | ○ | ● | ● | ● | + | – |
| | | | ○ | ● | ● | ● | + | + |
| VF | Dialyse | | | | | | – | – |
| | | | | | | | – | + |
| | | | | | | | + | – |
| | | | | | | | + | + |

**Fig. 1 C**

**Fig. 1 D**

**Fig. 2 A**

**Fig. 2 B**

**Fig. 3**

**A**

**Fig. 4 A**

**B**

**Fig. 4 B**

**Fig. 4 C**

**Fig. 5 A**

**Fig. 5 B**

← Zielprotein

★ Abbauprodukte

**Fig. 6**

Fig. 7

Fig. 8 A

**Fig. 8 B**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2006024781 A1
- DE 102013020900
- DE 102013015977

**In der Beschreibung aufgeführte Nicht-Patentliteratur**

- **CARLSON, E.D. et al.** *Biotechnology Advances,* 2012, vol. 30 (5), 1185-1194
- **MADIN, K. et al.** *Proc. Natl. Acad. Sci. USA,* 2000, vol. 97 (2), 559-64
- **SPIRIN, A. et al.** *Science,* 1988, vol. 242 (4882), 1162-4
- **CALLUS, B.A. ; D.L. VAUX.** Caspase inhibitors: viral, cellular and chemical. *Cell Death Differ,* 2006, vol. 14 (1), 73-78
- **EKERT, P.G. ; SILKE, J. ; VAUX, D.L.** Caspase inhibitors. *Cell Death Differ,* 1999, vol. 6, 1081-1086
- **KATZEN, F. ; G. CHANG ; W. KUDLICKI.** *Trends Biotechnol.,* 2005, vol. 23 (3), 150-156

SEQUENZ PROTOKOLL

<110> Fraunhofer-Gesellschaft
<120> Verfahren zur zellfreien Proteinsynthese unter Verwendung eines eukaryotischen Zelllysats in Gegenwart eines Caspase-Inhibitors, Vorrichtung zur Durchführung des Verfahrens und Verwendung eines Caspase-Inhibitors zur Steigerung der Proteinausbeute in einem solchen Verfahren
<140> PCT/EP
<141> 2014-09-17
<150> DE 10 2013 020 900.5
<151> 2013-12-11
<150> DE 10 2013 015 977.6
<151> 2013-09-25
<160> 10
<210> 1
<211> 4
<212> PRT
<213> Künstliche Sequenz
<220>
<223> Beschreibung: Caspase-Inhibitor
<400> 1

Asp Glu Val Asp
1

<210> 2
<211> 4
<212> PRT
<213> Künstliche Sequenz
<220>
<223> Beschreibung: Caspase-Inhibitor
<400> 2

Tyr Val Ala Asp
1

<210> 3
<211> 4
<212> PRT
<213> Künstliche Sequenz
<220>
<223> Beschreibung: Caspase-Inhibitor

<400> 3

Trp Glu His Asp

1

<210> 4
<211> 4
<212> PRT
<213> Künstliche Sequenz
<220>
<223> Beschreibung: Caspase-Inhibitor
<400> 4

Ala Glu Val Asp

1

<210> 5
<211> 4
<212> PRT
<213> Künstliche Sequenz
<220>
<223> Beschreibung: Caspase-Inhibitor
<400> 5

Leu Glu Glu Asp

1

<210> 6
<211> 5
<212> PRT
<213> Künstliche Sequenz
<220>
<223> Beschreibung: Caspase-Inhibitor
<400> 6

Val Asp Val Ala Asp

1                   5

<210> 7
<211> 4
<212> PRT
<213> Künstliche Sequenz
<220>
<223> Beschreibung: Caspase-Inhibitor
<400> 7

Val Glu Ile Asp

1

<210> 8
<211> 4
<212> PRT

<210> 9
<211> 4
<212> PRT
<213> Künstliche Sequenz
<220>
<223> Beschreibung: Caspase-Inhibitor
<400> 9

Ile Glu Thr Asp

1

<210> 9
<211> 4
<212> PRT
<213> Künstliche Sequenz
<220>
<223> Beschreibung: Caspase-Inhibitor
<400> 9

Leu Glu His Asp

1

<210> 10
<211> 20
<212> PRT
<213> Künstliche Sequenz
<220>
<223> Beschreibung: Caspase-Inhibitor
<400> 10

Ala Ala Val Ala Leu Leu Pro Ala Val Leu Leu Ala Leu Leu Ala
1               5                   10                  15
Pro Asp Glu Val Asp
                20